(19) Europäisches Patentamt
European Patent Office
Office européen des brevets

(11) Publication number: 0 034 062 B1

# (12) EUROPEAN PATENT SPECIFICATION

(45) Date of publication of patent specification: 06.04.83

(51) Int. Cl.³: C 07 C 69/16, C 07 C 67/00

(21) Application number: 81300516.2

(22) Date of filing: 06.02.81

## (54) Process for producing ethylidene diacetate.

(30) Priority: 08.02.80 JP 14601/80
08.04.80 JP 45884/80

(43) Date of publication of application:
19.08.81 Bulletin 81/33

(45) Publication of the grant of the patent:
06.04.83 Bulletin 83/14

(84) Designated Contracting States:
DE FR GB IT

(56) References cited:
BE - A - 879 178
US - A - 3 579 566

(73) Proprietor: MITSUBISHI GAS CHEMICAL COMPANY, INC.
5-2, Marunouchi 2-chome Chiyoda-Ku
Tokyo (JP)

(72) Inventor: Isshiki, Tomiya
14-11, Umegaoka 2-chome
Setagaya-ku Tokyo (JP)
Inventor: Ito, Akira
15-6, Nishikubo-cho
Tokiwadaira Matsudo-shi Chiba-ken (JP)
Inventor: Kijima, Yasuhiko
641, Koda
Matsudo-shi Chiba-ken (JP)
Inventor: Watanabe, Takayuki
6-1, Kanamachi 1-chome
Katsushika-ku Tokyo (JP)

(74) Representative: Cropp, John Anthony David et al,
MATHYS & SQUIRE 10 Fleet Street
London, EC4Y 1AY (GB)

EP 0 034 062 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for producing ethylidene diacetate

This invention relates to a process for producing ethylidenediacetate which comprises reacting acetic anhydride with hydrogen.

It is known to produce ethylidenediacetate by reducing acetic anhydride with hydrogen in the presence of cobalt or a noble metal belonging to Group VIII of the Periodic Table as a catalyst (see Japanese Patent Publication No. 19285/1973 and USP No. 3,579,566).

When cobalt is employed as a catalyst, carbon monoxide must be present in the reaction system. In addition, in separating the resulting reaction product by distillation, volatile cobalt compounds such as cobalt carbonyl are incorporated in the product, and this makes the process unsatisfactory from industrial point of view.

When noble metals belonging to Group VIII of the Periodic Table are employed, organic phosphine, organic arsine or organic stibine are employed with the noble metal. However, the organic phosphine, organic arsine and organic stibine are special materials and are expensive. As known by the specialists in this field, these organic compounds are readily oxidized in air to inactive materials in the reaction. In addition, on account of instability the organic compounds tend to lose co-ordinating ability within a relatively short time. Furthermore, since the life of these organic compounds, such as organic phosphine, organic arsine and organic stibine, is short, the industrial process employing these compounds is not economic. In addition, since these organic compounds are very toxic, the processing step employing these compounds must be controlled carefully so that the compounds are not incorporated in the object product.

Belgian patent specification 879178 proposes a process employing as the catalyst system an insoluble metal-based hydrogenation catalyst and a strong protonic acid such as hydrogen chloride, hydrogen fluoride or methane sulfonic acid. However, when a hydrogen halide is used in the catalyst system, significant quantities of haloethylidene acetate are formed as a by-product.

According to the present invention, there is provided a process for producing ethylidenediacetate which comprises reducing acetic anhydride with hydrogen in the presence of a catalyst comprising (a) as a main component at least one material selected from metals belonging to Group VIII of the Periodic Table, compounds of the metals and mixtures thereof and (b) as a secondary component (i) at least one alkyl halide selected from alkyl chlorides, alkyl iodides, alkyl bromides and mixtures thereof or (ii) at least one material selected from the group consisting of ammonia, organic nitrogen compounds and mixtures thereof; substantially anhydrous conditions being employed.

The mechanism of the reduction of acetic anhydride in this invention is not perfectly clear. However, it is believed that the reaction is expressed by the following equation:

$$2(CH_3CO)_2O+H_2 \rightarrow CH_3CH(OCOCH_3)_2+CH_3COOH$$

In synthesis of ethylidenediacetate according to this invention, at least one material selected from the group consisting of metals belonging to Group VIII of the Periodic Table, compounds of the metals or mixtures thereof is used as a main component constituting the catalyst, and at least one material selected from the group consisting of alkyl iodides, alkyl bromides, alkyl chlorides and mixtures thereof or at least one material selected from the group consisting of ammonia, organic nitrogen compounds and mixtures thereof is used as a secondary component constituting the catalyst.

The main components include metals belonging to Group VIII of the Periodic Table and compounds of these metals. These metals can be used in any form of zero valence state and any higher valence state. For example, they can be used as metal itself or Raney metals, or finely divided particles of the metals, or as metal compounds, such as carbonates, oxides, peroxides, hydroxides, nitrates, sulfates, phosphates, halides, cyanides, thiocyanides, sulfonates, $C_1$—$C_5$ alkoxides, such as methoxides and ethoxides, phenoxide, carboxylates derived from $C_1$—$C_{20}$ alkanoic acids, oxyhalides, hydrides, carbonyls, nitrites, sulfites, phosphites, acetylacetonates and sulfides of metals or metal compounds co-ordinated with ammonia, cyanide, amines or amino acids.

The main components include, for example, $Rh(OH)_3$, $Rh(NO_3)_3 \cdot 2H_2O$, RhO, $RhO_2$, $Rh_2O_3$, $Rh_2(SO_4)_3 \cdot 6H_2O$, RhS, $[Rh(AcO)_2]_2$, $Rh_2(CO)_3$, $[RhX(CO)_2]_2$, $Rh(AcAc)_3$, $Rh(SCN)_3$, $Rh(OPh)_3$, metallic Rh, $RhX_3$, $RhX_3 \cdot 3H_2O$, $Rh_6(CO)_{16}$, $RhX(CO)[P(n\text{-}C_4H_9)_3]_2$, $[Rh(C_2H_4)_2Cl]_2$, $[Rh(AcO)_2]_2$, $[Rh(CO)_2X][(n\text{-}C_4H_9)_4N]$, $K_4Rh_2X_2(SnX_3)_4$, $PdX_2$, $[Pd(CO)X_2]_2$, metallic palladium, $PdX_2$, $PdX_2 \cdot 2H_2O$, $PdX_2 \cdot 2NH_3$, $Pd(CN)_2$, $Pd_2H$, $Pd(OH)_2$, $Pd(OH)_2 \cdot 2NH_3$, $Pd(NO_3)_2$, $Pd_2O$, PdO, $PdO_2$, $PdSO_4 \cdot 2H_2O$, $Pd_2S$, PdS, $PdS_2$, $Pd_3(PO_4)_2$, $Na_2PdX_4$, $K_2PdX_4$, $Li_2PdX_4$, $Pd(OAc)_2$, $Pd(AcAc)_2$, $PdX_2(PhCN)_2$, Pd(SCN), $Pd(NC)_2$, palladium benzenesulfonate, $[PdX(C_3H_5)]_2$, $[Pd(CO)X_2]_2$, $PdX_2(C_8H_{12})$, metallic Pt, $H_2PtX_6$, $PtX_2$, $PtX_4$, $Pt(OH)_2$, $Pt(OH)_4$, $PtO_2$, PtO, $Pt_3O_4$, $Pt(CO)X_2$, PtS, $Pt_2S_3$, $Pt(CN)_2$, $H_2PtX_6$, $Pt(CO)X_2$, $[Pt(CO)X_3][(n\text{-}C_4H_9)_4N]$, $IrX_3$, $IrX_3 \cdot 3H_2O$, $Ir_2(CO)_4X_2$, $Ir_2(CO)_8$, $[Ir(CO)_2X_2][(n\text{-}C_4H_9)_4N]$, $[Ir(C_2H_4)_2Cl]_2$, $K_4Ir_2X_2(SnX_3)_4$, metallic Ir, $IrO_2$, $IrO_2 \cdot 2H_2O$, $Ir_2O_3 \cdot 3H_2O$, IrS, Metallic Ru, $RuX_2$, $RuX_4$, $Ru(OH)_3$, $RuO_2$, $Ru_2O_3$, $Ru(NO_3)_3 \cdot 6H_2O$, $Ru(CO)_2I_2$, $Ru_3(CO)_{12}$, $RuX_3$, $RuX_3 \cdot 3H_2O$, $Ru(CO)_2I_2$, $K_2[Ru_2(SnCl_3)_2]$, metallic Os, $OsX_2$, $OsX_3$, $Os(CO)_4X_2$, $Os_3(CO)_{12}$, $Os(CO)_5$, metallic Fe, $FeS_2$, $FeX_2$, $Fe(NO_3)_2 \cdot 6H_2O$ $FeSO_4 \cdot 5H_2O$, $Fe(H_2PO_2)_3$, $FeSO_3$, $FeS_2O_3 \cdot 5H_2O$, $Fe(NO_2)_2$, $Fe(CO)_5$, $H_2Fe(CO)_4$, metallic Ni, $NiX_2$,

$NiX_2 \cdot 3H_2O$, $Ni(CO)_4$, $Ni(CO)_2(PPh_3)_2$, $Ni(AcAc)_2$, Raney alloy, NiO, $Ni_2O_3$, $NiCO_3$, $NiSO_4$, NiS, $Ni(CN)_2$, $CoI_2$, $[Co(CO)_4]_2$, $HCo(CO)_4$, metallic Co, $Co_3O_4$, $CoX_3$, $Co(OAc)_2 \cdot 4H_2O$, $CoCO_3$, $Co_2(SO_4)_3$, $CoSO_4$, wherein X is F, Cl, Br or I, Ph is phenyl group, OPh is phenoxy group, AcO is acetoxy group and AcAc is acetylacetonate group. In addition, metal-polymer complexes as disclosed in CHEMTECH, 1973 Pages 560—566 and CHEMTECH, 1975 pages 117—122 can be used as a main component of the catalyst.

For example, the metal-polymer complexes are those in which a metal belonging to Group VIII of the Periodic Table is bonded to silica, polyvinyl chloride, or polystyrene-divinylbenzene substrate crosslinked by phosphine, silyl, an amine, pyridine or sulfido bond.

Of these metals and metal compounds, palladium, iridium, ruthenium, platinum, rhodium, nickel and cobalt and compounds of these metals are preferable; palladium, iridium, ruthenium, platinum and rhodium and compounds of these metals are more preferable; and palladium, platinum, rhodium and ruthenium and compounds of these metals are most preferable.

The main components of the catalyst may be soluble in a reaction solution. In this case a catalytic reaction is effected in a homogeneous system. The main component may be insoluble in the reaction solution or partially soluble in the solution. In this case the reaction is effected in a heterogeneous system. The heterogeneous system catalysts include, for example, a metal itself, compounds of the metals or a metal held on a carrier.

A metal can be held on a carrier by coprecipitation, impregnation, blending, adsorption, and ion exchange and the like.

One of the processes for holding a metal on a carrier is as follows: A carrier is impregnated with a solution of a metal belonging to Group VIII of the Periodic Table or a compound of the metal. The metal or the metal compound on the carrier is reduced with formalin, hydrogen, sodium formate, carbon monoxide, sodium borohydride, lithium aluminum hydride, or hydrazine, and then dried. Of course, the main component may be held on a carrier by other methods.

Examples of the carriers include carbon, graphite, bone black, alumina, silica, silica alumina, barium sulfate, natural or synthetic zeolite, spinel, magnesia adhered alumina, thoria, titanium oxide, zirconium oxide, thorium oxide, lanthanum oxide, cerium oxide, zinc oxide, tantalum, clay, diatomaceous earth, Celite (trade name of product being commercially available from Johns-Manville Co.), asbestos, pumice stone, bauxite, terra alba, natural and treated terra alba, such as Super-Filtrol, silicon carbite, molecular sieves, ceramic honeycomb, boria, cements, alundum, corundum, brick, talc and gypsum. Carbon, graphite, bone black, alumina, silica, silica alumina, barium sulfate, zeolite spinel and magnesia adhered alumina are preferred. The carriers may be in the form of particles having uniform or ununiform size. The carriers may be in the form of molded articles, extruded articles, ceramic bars, balls, fragments and tiles.

Heterogeneous system catalysts are preferred to homogeneous system catalysts, because separation and purification of the product or separation and recovery of the catalyst are easy in a heterogeneous system.

Either one of (i) at least one alkyl halide selected from alkyl chlorides, alkyl bromides, alkyl iodides and mixtures thereof or (ii) at least one material selected from ammonia and organic nitrogen compounds may be used as a secondary component constituting the catalyst. Alkyl halides are preferable as a secondary component.

Methyl chloride, methyl bromide and methyl iodide are more preferable, because the use of these compounds makes easy selection of corrosion-resistant reactor and separation of the reaction product from the reaction mixture and purification of the reaction product.

Materials which can convert to an alkyl halide can also be used as a secondary component.

Ammonia or organic nitrogen compounds employed as a secondary component (ii) are capable of forming a co-ordination compound with the main component having one or more electron pairs being capable of forming a co-ordination bond with the metal in the main components. The secondary component (ii) may be introduced to the reaction zone together with the reactants. Alternatively the secondary component (ii) may first be bonded to a main component and then the resulting metal complex may be introduced to the reaction zone.

Examples of ammonia and organic nitrogen compounds are as follows:

(I) Trivalent nitrogen compounds

(A) compounds represented by the formula

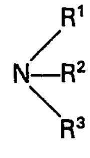

(a) wherein $R^1$, $R^2$ and $R^3$ may be the same or different, and in dependently hydrogen, alkyl having 1—10 carbon atoms, cycloalkyl having 3—10 carbon atoms or aryl; the compounds include, for example ammonia and amines, such as monomethyl amine, dimethyl amine, trimethyl amine,

monoethyl amine, diethyl amine, triethyl amine, dimethyl ethyl amine, tri-n-propyl amine, tri-isopropyl amine, tri-n-butyl amine, tri-tert.-butyl amine, aniline, dimethyl aniline, diethyl aniline, dimethylbenzyl amine, toluidine, and cyclohexyl amine:

(b) wherein $R^1$ is hydrogen, alkyl having 1—10 carbon atoms, cycloalkyl having 3—10 carbon atoms, or aryl and $R^2$ and $R^3$ are taken together and represents methylene or polymethylene having 2—5 carbon atoms; the compounds include, for example pyrrolidine, N-methyl pyrrolidine or piperidine or N-phenyl piperidine:

(c) wherein $R^1$ and $R^2$ may be the same or different and independently represent hydrogen, alkyl having 1—10 carbon atoms, cycloalkyl having 3—10 carbon atoms, or aryl and $R^3$ is aliphatic saturated acyl, or $R^1$ is hydrogen, alkyl having 1—10 carbon atoms, cycloalkyl having 3—10 carbon atoms or aryl, and $R^2$ and $R^3$ are taken together and represent lactam ring; the compounds include, for example carboxylamines such as acetamide, N,N-dimethylacetamide, acetoanilide, N-methyl-N-phenylacetamide, and lactam, such as N-methyl-pyrrolidinone:

(d) wherein at least one of $R^1$, $R^2$ and $R^3$ is carboxymethyl and the remainder is hydrogen, alkyl having 1—10 carbon atoms, cyclohalkyl having 3—10 carbon atoms or aryl; the compounds include, for example carboxylic acid derivatives, such as N,N-dimethyl glycine, N,N-diethyl glycine, imino diacetic acid, N-methyl iminodiacetic acid, or nitrilotriacetic acid:

(B) Organic compounds represented by the formula N≡C—R wherein R represents an alkyl having 1 to 10 carbon atoms, a cycloalkyl or an aryl; the compounds include for example nitriles, such as acetonitrile, propionitrile, or benzonitrile.

(C) Organic nitrogen compounds represented by the formula

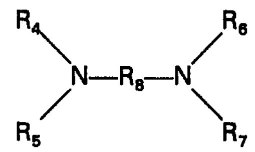

(a) wherein $R^4$, $R^5$, $R^6$ and $R^7$ may be the same or different and independently hydrogen, alkyl having 1—10 carbon atoms, cycloalkyl having 3—10 carbon atoms, or aryl and $R^8$ is methylene group or polymethylene group having 2—10 carbon atoms, phenylene, or carbonyl group; the compounds include, for example N,N,N',N'-tetramethylethylenediamine, N,N,N',N'-tetraethylethylenediamine, N,N,N',N'-tetra-n-propylethylenediamine, N,N,N',N'-tetramethylmethylenediamine, N,N,N',N'-tetramethyl urea or N-methyl-pyrrodinone.

(b) $R^4$ and $R^6$ may be the same or different and independently represent hydrogen, alkyl having 1—10 carbon atoms, cycloalkyl having 3—10 carbon atoms or aryl and $R^5$ and $R^7$ are taken together and represent methylene or polymethylene having 2—5 carbon atoms and $R^8$ is methylene or polymethylene having 2—5 carbon atoms; the compounds include for example piperazine, N-methyl-piperazine, N-ethyl-piperazine, or 2-methyl-N,N'-dimethylpiperazine.

(c) other compounds include, for example 2,5-dicarboxypiperazine, cyclohexane-1,2-diamine N,N,N',N'-tetraacetic acid or salts thereof, tetramethylester, ethylenediaminetetraacetic acid salts or tetramethylester thereof, 1,4-azabicyclo[2,2,2]octane, methyl substituted, 1,4-diazabicyclo[2,2,2]octane, adiponitrile or N-methylmorpholine.

(II) Heterocyclic compounds include, for example, pyrrole; pyrrolines; pyrimidine; pyrazine; pyrazole; pyrazoline; pyridazine; picolines, such as α-picoline, β-picoline or γ-picoline; lutidines, such as 2,6-lutidine, 2,4-lutidine; collidines, such as 2,4,6-collidine; benzotriazole; dipyridyls, such as 2,2'-dipyridyl, 4,4'-dimethyl-2,2'-dipyridyl, or 4-triethylsilyl-2,2'-dipyridyl; pyridines such as pyridine, α-aminopyridine, β-aminopyridine, 2-hydroxypyridine, methyl-substituted-2-hydroxypyridine, picolinic acid, methyl-substituted picolinic acid, 2,6-dicarboxypyridine, 2-(dimethylamino)pyridine; imidazole; phenanthrolines, such as 1,10-phenanthroline, 4-chloro-1,10-phenanthroline, or 5-(thiabentyl)-1,10-phenanthroline; quinolines, such as quinoline, 2-(dimethylamino)-6-methoxyquinoline, 8-hydroxyquinoline or 2-carboxyquinoline.

(III) Pentavalent nitrogen compounds such as ammonium acetate, ammonium propionate, triammonium phosphate, diammonium hydrogenphosphate or ammonium dihydrogenphosphate.

Rate of the reduction of acetic anhydride depends on amount of the main component employed, and concentration of the secondary component.

Amount of the main component employed depends on type of the catalyst employed, such as homogeneous system or heterogeneous system, and type of the reaction whether being effected in a fixed bed or fluidized bed. However, in general, the amount of the main component employed may be in the range of from $1\times10^{-4}$ to 25 wt%, preferably in the range of from $5\times10^{-4}$ to 20 wt% more preferably from $1\times10^{-3}$ to 15 wt% and most preferably from $2.5\times10^{-3}$ to 10 wt% on the basis of weight of a reaction solution in terms of metal.

Amount of alkyl halides employed as secondary components employed may be in the range of from $10^{-3}$ to 15 mol, preferably $10^{-2}$—5 mol and preferably $10^{-1}$—3 mol per 1 liter of a reaction solution in terms of halogen atom.

Amount of ammonia or an organic nitrogen compound employed as a secondary component may be in the range of from $10^{-6}$ to 10 mol, preferably $10^{-4}$ to 5 mol and preferably $10^{-3}$ to 1 mol per 1 liter of a reaction solution.

In practising this invention, the reaction temperature is not critical. In general, the reaction temperature may be in the range of 20°C—500°C, preferably 30—350°C, more preferably 40—250°C.

The reaction pressure is kept high enough to keep the raw material(s), the solvent and the product in a liquid phase and to maintain an appropriate partial pressure of hydrogen. The partial pressure of hydrogen may be in the range of 0.2—350 atm. (0.2—350 bar) preferably 0.5—300 atm. (0.5—300 bar) and more preferably 1—200 atm. (1—200 bar). However, partial pressure of hydrogen may be in the range of 0.05—1000 atm. (0.05—1000 bar).

Hydrogen used as a starting material need not necessarily have high purity. The hydrogen may contain carbon monoxide, carbon dioxide, methane, nitrogen, rare gases and the like. Synthesis gas mixture of hydrogen and carbon monoxide may be used as a raw material. Carbon monoxide contained in the hydrogen tends to make the catalyst stable and to restrain side reactions and consequently assists the reaction to proceed effectively. However, hydrogen of extremely low purity is not preferred, because other gases contained in the hydrogen increase the pressure of reaction system. Therefore, hydrogen with purity below 2 mol % is not preferred as a raw material. In general, hydrogen with purity above 5 mol % may be used, and preferably hydrogen with purity above 10 mol % may be used.

Acetic anhydride employed as a raw material may be prepared as follows:

Acetic anhydride may be prepared by oxidization of acetaldehyde or via ketene (so-called Wacker process) from acetic acid which may be prepared by reaction of methanol and carbon monoxide. Alternatively acetic anhydride may be prepared from methyl acetate and carbon monoxide. As mentioned above acetic acid, methyl acetate and acetaldehyde are incorporated in acetic anhydride employed as a raw material. However, incorporation of these compounds in acetic anhydride is permitted, as long as an overall balance of the process is obtained.

A large amount of water is preferably avoided in this process because such large amount of water causes decomposition of raw materials and products. However, since commercially available hydrogen and acetic anhydride contain small amounts of water, hydrogen and acetic anhydride containing water of such low concentration are permitted in this invention.

By "substantially anhydrous conditions" is meant that water content in the reaction system is less than 10 mol % on the basis of total mol of the liquid components present in the system. In general, water content less than 5 mol % is preferable, and water content less than 3 mol % is more preferable. When raw materials contain a large amount of water, they should be dried before introducing them into the reaction system.

Since acetic anhydride acts as a solvent for the reaction, another solvent is not necessary but as occasion demands, another solvent may be used. Examples of solvents include organic acids, such as acetic acid, propionic acid, butyric acid, octanoic acid, phthalic acid, benzoic acid; esters of organic acids, such as methyl acetate, ethyl acetate, ethylene glycol diacetate, propylene glycol diacetate, dimethyl adipate, methyl benzoate, ethyl benzoate, dimethyl phthalate, diethyl phthalate, dioctyl phthalate, phenyl acetate, and tolyl acetate; hydrocarbons, such as dodecane, hexadecane, benzene, naphthalene, and biphenyl; esters of inorganic acids, such as triphenyl phosphate, tricresyl phosphate, dibutylphenyl phosphate; silicates, such as tetramethyl ortho-silicate, and tetrabutyl silicate; aromatic ethers, such as diphenyl ethers, ketones, such as acetone, methyl ethyl ketone, dibutyl ketone, methyl isobutyl ketone, acetophenone, and benzophenone. Use of acetic acid or carboxylic esters, such as methyl acetate or mixture thereof is preferred as a solvent.

The present process may be carried out by batch, semi-continuous or continuous method. When a heterogeneous system catalyst is used, the reaction can be effected in a fluidized bed or a fixed bed. In continuous process, the raw material and the catalyst are continuously introduced into the reaction zone; reduction of acetic anhydride with hydrogen is effected; and the reaction mixture is continuously removed from the zone. Ethylidenediacetate is separated from the reaction mixture, for example by distillation.

In another embodiment, the reaction mixture containing ethylidenediacetate removed from the reaction zone is introduced into another reaction zone as it is, or alternatively after part of the components other than ethylidenediacetate are separated from the reaction mixture, the resulting reaction mixture may be introduced into another reaction zone. In the reaction zone, ethylidenediacetate may be converted to acetic acid and vinyl acetate. From the resulting mixture acetic anhydride, acetic acid and vinyl acetate may be separated by distillation, and vinyl acetate may be obtained as the product.

Acetic acid obtained in this process may be esterified with methanol to form methyl acetate. The methyl acetate so obtained may be reacted with carbon monoxide to form acetic anhydride. The resulting acetic anhydride can be used in the present invention.

A process for producing ethylidenediacetate from acetic anhydride by using a novel catalyst is very advantageous from commercial point of view.

The following examples are given as illustrative embodiments of the invention and should not be construed as limiting its scope.

All parts and percents are on weight, unless otherwise specified.

Example 1

Into an autoclave were charged 40.0 g of acetic anhydride, 10.0 g of methyl iodide, and 1.5 g of palladium (5%) held activated carbon being commercially available from Nippon Engelhard. Hydrogen (80 $Kg/cm^2$, 80 bar) under pressure was charged into the autoclave.

The temperature in the autoclave was raised to 175°C, and the reaction was continued for 4 hours. During the reaction, the pressure in the autoclave was decreased considerably. After cooling the reaction mixture, GC analysis showed that it contained 21.3 g of ethylidenediacetate, 0.013 g of acetaldehyde, 0.24 g of ethyl acetate and 0.55 g of methyl acetate. The remainder components were unreacted acetic anhydride, methyl iodide and acetic acid.

Example 2

Alumina-magnesia (alumina:magnesia=9:1) having 100—200 mesh which is commercially available from Mizusawa Chemical KK was washed with an NaOH solution and water. A palladium chloride solution in hydrochloric acid was added to the alumina-magnesia so treated. The mixture was stirred at 80°C for a given time. After pH of the solution was adjusted to 9—10 by adding water and an NaOH solution thereto, palladium compound on the alumina-magnesia was reduced with formalin. After pH of the solution was adjusted to 7 by adding hydrochloric acid thereto, the palladium-adhered alumina-magnesia was removed from the solution, and dried to obtain palladium-held alumina-magnesia. Amount of palladium held was 1%.

Into an autoclave were charged 40.0 g of acetic anhydride, 5.0 g of methyl iodide and 6.75 g of palladium (1%)-held alumina-magnesia. Hydrogen (80 $Kg/cm^2$, 80 bar) under pressure was charged into the autoclave. The temperature in the autoclave was raised to 175°C and the reaction was continued for 1 hr. After cooling the reaction mixture, GC analysis showed that it contained 11.7 g of ethylenediacetate, 1.31 g of acetaldehyde, 0.231 g of ethyl acetate and 0.756 g of methyl acetate. Other components were unreacted acetic anhydride, methyl iodide and acetic acid.

Example 3

Into a autoclave were charged 40.0 g of acetic anhydride, 10.0 g of methyl iodide and 1.5 g of palladium (5%)-held activated carbon. Mixed gas of $H_2$ and CO ($H_2$:CO=8:2 by volume) (100 $Kg/cm^2$, 100 bar) under pressure was charged. The temperature in the autoclave was raised to 175°C, and the reaction was continued for 4 hours. After reacting, GC analysis showed that it contained 24.6 g of ethylidenediacetate, 0.41 g of acetaldehyde, 0.34 g of ethyl acetate and 0.30 g of methyl acetate.

Comparative Experiment 1

The procedure of Example 3 was repeated except that 51.0 g of acetic anhydride is used and methyl iodide is not used. GC analysis showed that ethylidenediacetate was not formed.

Examples 4—17

Acetic anhydride, main components and secondary components were charged into an autoclave as shown in Table 1. Hydrogen or mixed gas of $H_2$ and CO under pressure was charged into the autoclave as shown in Table 1. The results are shown in Table 1. The reaction pressure in Examples 16 and 17 was maintained at 30 $Kg/cm^2$ (30 bar) during the reaction by introducing hydrogen continuously. The pressure in Examples 4—15 was that under which hydrogen or mixed gas was introduced. In these Examples after the reaction started, hydrogen was not introduced. The main components employed in Examples 4, 7, 9, 11, 14, 15, 16 and 17 were commercially available from Nippon Engelhard KK. The main components employed in Examples 6, 12 and 13 were commercially available from Kawaken Fine Chemical KK.

TABLE 1

| Ex. No. | Acetic anhydride (g) | Main component (g) | | Halide (g) | Gas ($H_2$:CO) | Pressure ($kg/cm^2$) (bar) | Tempera-ture (°C) | Reaction time (Hr) | Product | |
|---|---|---|---|---|---|---|---|---|---|---|
| | | | | | | | | | Ethylidene-diacetate (g) | Acetald-hyde (g) |
| 4 | 40 | Palladium black | 0.075 | $CH_3I$ 10 | 8:2 | 100 | 175 | 4 | 21.7 | 0.014 |
| 5 | 40 | Palladium acetate | 0.158 | $CH_3I$ 10 | 8:2 | 100 | 175 | 4 | 20.8 | — |
| 6 | 40 | 5% Pd/$BaSO_2$ | 1.5 | $CH_3I$ 10 | 8:2 | 100 | 175 | 5 | 15.2 | — |
| 7 | 40 | 5% Rh/activated C | 1.5 | $CH_3I$ 10 | 8:2 | 100 | 175 | 3 | 7.31 | — |
| 8 | 40 | rhodium acetate | 0.204 | $CH_3I$ 10 | 8:2 | 100 | 175 | 4 | 10.2 | 0.08 |
| 9 | 40 | 5% Ru/activated C | 1.5 | $CH_3I$ 10 | 10:0 | 80 | 175 | 4 | 5.49 | — |
| 10 | 40 | ruthenium chloride | 0.194 | $CH_3I$ 10 | 8:2 | 100 | 175 | 4 | 5.34 | — |
| 11 | 40 | 5% Pt/activated C | 1.5 | $CH_3I$ 10 | 10:0 | 80 | 175 | 6 | 2.87 | — |
| 12 | 40 | Raney Ni | 0.5 | $CH_3I$ 10 | 10:0 | 80 | 175 | 5 | 1.15 | — |
| 13 | 40 | Raney Co | 0.4 | $CH_3I$ 10 | 10:0 | 80 | 175 | 8 | 0.131 | — |
| 14 | 40 | 5% Pd/activated C | 1.25 | $CH_3C$ 15.35 | 10:0 | 80 | 175 | 9 | 15.2 | — |
| 15 | 40 | 5% Pd/activated C | 1.63 | $CH_3Br$ 2.0 | 10:0 | 80 | 175 | 2 | 26.3 | 1.02 |
| 16 | 40 | 5% Pd/activated C | 1.35 | $CH_3I$ 5.0 | 10:0 | 30 | 175 | 3 | 28.7 | 0.16 |
| 17 | 40 | 5% Pd/activated C | 1.28 | $CH_3I$ 2.5 | 10:0 | 30 | 175 | 4 | 26.8 | 0.35 |

Example 18

Into an autoclave equipped with blades for agitation and filter were charged 4.5 g of palladium (5%)-held activated carbon employed in Example 1, 120 g of acetic anhydride and 15 g of methyl iodide. The reaction was effected at 175°C at 50 $Kg/cm^2$ for 1 hour by introducing hydrogen into the system continuously. Thereafter, hydrogen (50 $Kg/cm^2$, 50 bar), and mixture of 89% acetic anhydride and 11% methyl iodide (135 g/hr) were introduced into the system continuously and at the same time the reaction mixture (135 g/hr) was continuously removed from the system through filter. During the reaction the temperature was at 175°C and the pressure was maintained at 50 $Kg/cm^2$ (50 bar) by introducing hydrogen. The reaction was continued for 100 hrs. Ethylidenediacetate (586 g) was recovered from the effluent by distillation.

Example 19

Into a 100 ml autoclave made of tantalum were charged 35.0 g of acetic anhydride, 0.265 g of rhodium chloride and 0.435 g of 2,6-lutidine. Air in the autoclave was purged with nitrogen. Hydrogen (120 $Kg/cm^2$, 120 bar) and carbon monoxide (40 $Kg/cm^2$, 40 bar) under pressure were charged into the autoclave. The temperature in the autoclave was raised to 175°C. The reaction was continued for 4 hours with stirring. The reaction pressure was lowered by 30 $Kg/cm^2$ (30 bar) during the reaction. After cooling the reaction mixture, GC analysis showed that it contained 6.31 g of ethylidenediacetate, 2,49 g of ethyl acetate and 9.33 g of acetic acid. The remainder was for the most part unreacted acetic anhydride.

Example 20

The procedure of Example 19 was repeated except that 0.224 g of palladium acetate was used in place of rhodium chloride. GC analysis showed that the reaction mixture contained 4.56 g of ethylidenediacetate, 1.86 g of ethyl acetate and 6.37 g of acetic acid.

Example 21

The procedure of Example 19 was repeated except that 0.314 g of ruthenium chloride was used in place of rhodium chloride and 0.400 g of 2,6-lutidine was used. GC analysis showed that the reaction mixture contained 1.70 g of ethylidenediacetate and 6.04 g of ethyl acetate.

Example 22

The procedure of Example 19 was repeated except that carbon monoxide was not used. GC analysis showed that the reaction mixture contained 5.87 g of ethylidenediacetate.

Example 23

The procedure of Example 21 was repeated except that carbon monoxide was not used. GC analysis showed that the reaction mixture contained 1.56 g of ethylidenediacetate.

Examples 24—33

The reactions were effected under the conditions as shown in Table 2. The partial pressure of hydrogen to that of carbon monoxide was 3:1. The results are shown in Table 2.

TABLE 2

| Ex. No. | Main component | Nitrogen compound | Initial pressure ($kg/cm^2$) (bar) | Temperature °C | Time Hrs | Ethylidene-diacetate g |
|---|---|---|---|---|---|---|
| 24 | rhodium chloride 0.530 g | 2,4-lutidine 1.31 g | 160 | 180 | 4 | 13.7 g |
| 25 | rhodium chloride 0.265 g | tri-n-butyl-amine 1.85 g | 80 | 175 | 4 | 4.17 g |
| 26 | rhodium chloride 0.265 g | triethylamine 0.01 g | 160 | 175 | 4 | 3.91 g |
| 27 | rhodium chloride 0.395 g | N,N-dimethyl formamide 0.10 g | 120 | 190 | 6 | 2.02 g |
| 28 | rhodium chloride 0.265 g | N,N,N',N'-tetramethyl-ethylidenediamine 0.570 g | 160 | 175 | 4 | 3.84 g |
| 29 | rhodium chloride 0.395 g | ammonium acetate 1.16 g | 160 | 200 | 6 | 2.82 g |
| 30 | palladium acetate 0.224 g | tri-n-butyl amine 2.78 g | 160 | 175 | 6 | 1.28 g |
| 31 | palladium chloride 0.213 g | pyridine 0.791 g | 160 | 175 | 4 | 4.63 g |
| 32 | palladium chloride 0.213 g | 2-hydroxy pyridine 0.951 g | 160 | 175 | 4 | 4.41 g |
| 33 | palladium chloride 0.213 g | benzonitrile 0.860 g piperidine 0.426 g | 160 | 175 | 7 | 0.86 g |

**Claims**

1. A process for producing ethylidenediacetate by the reduction of acetic anhydride with hydrogen under substantially anhydrous conditions in the presence of a catalyst comprising a metal component and a secondary component characterised in that as the metal component there is used at least one material selected from metals belonging to Group VIII of the Periodic Table, compounds of said metals and mixtures thereof and as a secondary component there is used (i) at least one alkyl halide selected from alkyl chlorides, alkyl bromides, alkyl iodides and mixtures thereof or (ii) at least one material selected from ammonia, organic nitrogen compounds and mixtures thereof.

2. The process as defined in Claim 1 characterised in that the metal component of the catalyst is selected from palladium, rhodium, iridium, ruthenium, platinum, nickel and cobalt and compounds of these metals.

3. The process as defined in Claim 1 or Claim 2 characterised in that the amount of the metal component employed is in the range of from $1 \times 10^{-4}$ to 25 wt% on the basis of weight of reaction solution in terms of metal.

4. The process as defined in any one of Claims 1 to 3 characterised in that the secondary component of the catalyst is selected from alkyl halides.

5. The process as defined in Claim 4 characterised in that the amount of halide employed as secondary component is in the range of from $10^{-3}$ to 15 mol per liter of reaction solution in terms of halogen atom.

6. The process as defined in any one of Claims 1 to 3 characterised in that the secondary component of the catalyst is selected from ammonia and organic nitrogen compounds.

7. The process as defined in Claim 6 characterised in that the amount of ammonia or organic nitrogen compound employed as secondary component is in the range of from $10^{-6}$ to 10 mol per liter of reaction solution.

8. The process as defined in any one of Claims 1 to 7 characterised in that the reaction pressure is kept high enough to keep the raw material and the product and optional solvent in a liquid phase.

9. The process as defined in any one of Claims 1 to 8 characterised in that the reaction is effected at a temperature in the range of from 20°C to 500°C.

**Patentansprüche**

1. Verfahren zur Herstellung von Ethylidendiacetat durch Reduktion von Essigsäureanhydrid mit Wasserstoff unter praktische wasserfreien Bedingungen in Gegenwart eines Katalysators mit einer Metallkomponente und einer zweiten Komponente, dadurch gekennzeichnet, daß der Katalysator als Metallkomponente mindestens ein Metall der Gruppe VIII des Periodensystems und/oder eine Verbindung solcher Metalle und als zweite Komponente 1. mindestens ein Alkylhalogenid in Form eines Alkylchlorids, Alkylbromids und/oder Alkyliodids oder 2. Ammoniak und/oder mindestens eines organische Stickstoffverbindung enthält.

2. Verfahren nach Anspruch 1, dadurch gekennzeichnet, daß der Katalysator als Metallkomponente Palladium, Rhodium, Iridium, Ruthenium, Platin, Nickel und/oder Kobalt und/oder Verbindungen dieser Metalle enthält.

3. Verfahren nach Ansprüchen 1 oder 2, dadurch gekennzeichnet, daß der Katalysator, bezogen auf das Gewicht der Reaktionslösung $1 \times 10^{-4}$ bis 25 Gew.-% Metallkomponente (ausgedrückt als Metall) enthält.

4. Verfahren nach einem der Ansprüche 1 bis 3, dadurch gekennzeichnet, daß der Katalysator als zweite Komponente mindestens ein Alkylhalogenid enthält.

5. Verfahren nach Anspruch 4, dadurch gekennzeichnet, daß der Katalysator, bezogen auf 1 l Reaktionslösung, $10^{-3}$ bis 15 Mol(e) Alkylhalogenid (ausgedrückt als Halogenatom) als zweite Komponente enthält.

6. Verfahren nach einem der Ansprüche 1 bis 3, dadurch gekennzeichnet, daß der Katalysator als zweite Komponente Ammoniak und/oder mindestens eine organische Stickstoffverbindung enthält.

7. Verfahren nach Anspruch 6, dadurch gekennzeichnet, daß der Katalysator, bezogen auf 1 l Reaktionslösung $10^{-6}$ bis 10 Mol(e) Ammoniak und/oder organische Stickstoffverbindung(en) als zweite Komponente enthält.

8. Verfahren nach einem oder mehreren der Ansprüche 1 bis 7, dadurch gekennzeichnet, daß der Reaktionsdruck hoch genug gehalten wird, um das Ausgangsmaterial und das Endprodukt sowie das gegebenenfalls vorhandene Lösungsmittel in flüssiger Phase zu halten.

9. Verfahren nach einem oder mehreren der Ansprüche 1 bis 8, dadurch gekennzeichnet, daß die Umsetzung bei einer Temperatur von 20° bis 500°C durchgeführt wird.

**Revendications**

1. Procédé de production de diacétate d'éthylidène par la réduction d'anhydride acétique avec de l'hydrogène en conditions sensiblement anhydres en présence d'un catalyseur comprenant un composant métallique et un composant secondaire, caractérisé en ce que, comme composant métallique, on utilise au moins un matériau choisi parmi des métaux appartenant au groupe VIII de la Table Périodique, des composés desdits métaux et leurs mélanges et comme composant secondaire, on utilise (i) au moins un halogénure d'alcoyle choisi parmi des chlorures d'alcoyle, des bromures d'alcoyle, des iodures d'alcoyle et leurs mélanges ou (ii) au moins un matériau choisi parmi l'ammoniac, des composés d'azote organique et leurs mélanges.

2. Procédé selon la revendication 1, caractérisé en ce que le composant de métal du catalyseur est choisi parmi le palladium, le rhodium, l'iridium, le ruthénium, le platine, le nickel, et le cobalt et des composés de ces métaux.

3. Procédé selon l'une quelconque des revendications 1 ou 2, caractérisé en ce que la quantité du composant de métal employé est comprise entre $1 \times 10^{-4}$ et 25% en poids sur la base du poids de la solution réactionnelle en termes du métal.

4. Procédé selon l'une quelconque des revendications 1 à 3, caractérisé en ce que le composant secondaire du catalyseur est choisi parmi des halogénures d'alcoyle.

5. Procédé selon la revendication 4, caractérisé en ce que la quantité de l'halogénure employé

comme composant secondaire est comprise entre $10^{-3}$ et 15 moles par litre de la solution de réaction en termes de l'atome d'halogène.

6. Procédé selon l'une quelconque des revendications 1 à 3, caractérisé en ce que le composant secondaire du catalyseur est choisi parmi l'ammoniac et des composés d'azote organique.

7. Procédé selon la revendication 6, caractérisé en ce que la quantité d'ammoniac ou du composé d'azote organique employé comme composant secondaire est comprise entre $10^{-6}$ et 10 moles par litre de la solution de réaction.

8. Procédé selon l'une quelconque des revendications 1 à 7, caractérisé en ce que la pression de la réaction est maintenu suffisamment élevée pour maintenir la matière première et le produit et le solvant éventuel en phase liquide.

9. Procédé selon l'une quelconque des revendications 1 à 8, caractérisé en ce que la réaction est effectuée à une température comprise entre 20 et 500°C.